(19) Europäisches Patentamt
European Patent Office
Office européen des brevets

(11) **EP 4 545 029 B1**

(12) **EUROPEAN PATENT SPECIFICATION**

(45) Date of publication and mention
of the grant of the patent:
**17.06.2026 Bulletin 2026/25**

(21) Application number: **24208289.9**

(22) Date of filing: **23.10.2024**

(51) International Patent Classification (IPC):
*A61B 18/12* (2006.01)    *A61B 18/14* (2006.01)
*A61B 18/00* (2006.01)

(52) Cooperative Patent Classification (CPC):
**A61B 18/1206;** A61B 18/1402; A61B 2018/1273;
A61B 2018/128

(54) **ELECTROSURGICAL GENERATOR WITH DUAL OUTPUT**

ELEKTROCHIRURGISCHER GENERATOR MIT DOPPELTEM AUSGANG

GÉNÉRATEUR ÉLECTROCHIRURGICAL À DOUBLE SORTIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **27.10.2023 DE 102023129686
27.10.2023 US 202363546035 P**

(43) Date of publication of application:
**30.04.2025 Bulletin 2025/18**

(73) Proprietor: **Olympus Winter & Ibe GmbH
22045 Hamburg (DE)**

(72) Inventors:
• **Wange Pinto, Isabel
12277 Berlin (DE)**

• **Janich, Fabian
14469 Potsdam (DE)**
• **Ramin, Daniel
14558 Nuthetal (DE)**
• **Fähsing, Thomas
12305 Berlin (DE)**

(74) Representative: **Glawe, Delfs, Moll
Partnerschaft mbB
Hopfenmarkt 33
20457 Hamburg (DE)**

(56) References cited:
EP-A1- 3 912 580    EP-A1- 4 389 043
US-A1- 2022 370 115

**Description**

The invention relates to an electrosurgical generator configured to output a high-frequency alternating voltage to electrosurgical instruments. The electrosurgical generator comprises a generation unit generating high-frequency (HF) alternating voltages to be supplied to at least two output sockets configured for simultaneous connection and activation of electrosurgical instruments.

In electrosurgery or high-frequency surgery, an electrosurgical instrument such as an electroscalpel is used to apply high-frequency alternating current to tissue in the human body. Usually high-frequency in the radiofrequency range of about 200 kHz to up to 4,000 kHz is used. This results in local heating of the tissue. Thereby, the tissue is cut or severed by heating, and the tissue is removed by thermal resection. A major advantage of this is that bleeding can be stopped at the same time as the cutting is made by closing the affected vessels, and electrosurgical instruments can be used for other applications, such as coagulation. Different kind of applications require different electrosurgical instruments.

For some applications it may be required to perform surgery tasks utilizing more than one, in particular two electrosurgical instruments at the same time (dual activation). In order to make this feasible, separate electrosurgical generators may be used, one for each electrosurgical instrument. A typical problem encountered is that interference between the outputs of the high-voltages generated by the generators may occur. Such interference may include an unwanted low-frequency beat similar to the phenomenon as encountered in the field of acoustics if two frequencies are close to each other but do not exactly match. Further, cross-currents in the body develop which flow across from one of the electrosurgical instrument to the other. Such cross-currents hamper proper control of the currents/power delivered to either electrosurgical and are thus adverse to operational safety. Apart from this, another costly difficulty is the need of having to provide two electrosurgical generators.

A similar situation may be encountered if a single electrosurgical generator having dual inverters and output stages is employed, thereby allowing dual activation of electrosurgical instruments. However, avoiding unwanted interference is still difficult. This may be alleviated by employing a single inverter supplying the two outputs in an alternating manner. However, this limits usage to modulated modes having a duty cycle that is smaller than 50%, which is a substantial restriction.

It was further envisaged to perform a power measurement over both outputs. This, however, has a drawback of being feasible only for outputs having the same settings and mode. Further, the true power distribution between each of these dual outputs will still be unknown.

Another approach is to supply the dual outputs with voltages having two different fundamental frequencies and to perform a discrete Fourier transformation (DFT) on measurements on both outputs using filter of a Goertzel type (EP 3 912 580 A1). If the fundamental frequencies are not completely orthogonal there will be overlap between them, said overlap producing discontinuities in a Goertzel array plot. This is used to detect whether significant cross-conductance occurs. If a certain threshold is exceeded, then HF generation and output will be shut down as a safety measure. As a further variant, based on this approach a beat amplitude can be measured in order to determine a magnitude of impedance. However, this allows for an estimation of the cross-current at best and is not enabled for a precise determination of the working current which is the current actually injected into the worked tissue by the respective electrosurgical instrument connected to either output. Further, it is known to reduce cross-currents (US 2022/370115 A1). As a result, although this may be a viable safety device, control of energy delivered by the respective electrosurgical instrument still needs improvement.

It is thus an object of the invention to provide an improved electrosurgical generator having dual outputs that lessens this drawback.

The solution according to the invention resides in the features of the independent claim. Advantageous embodiments are the subject matter of the dependent claims.

An electrosurgical generator configured to output a high-frequency alternating voltage to electrosurgical instruments for working on a tissue, comprising a first HF generation unit supplying a first high-frequency alternating voltage having a first frequency to a first output, a second HF generation unit supplying a second high-frequency alternating voltage having a second frequency which is different from the first frequency to a second output, the first and second output being configured for connection of a first and second electrosurgical instrument such that in an activated state a first total current flows to the first electrosurgical instrument and simultaneously a second total current flows to the second electrosurgical instrument, and a central control unit configured to control operation of the electrosurgical generator including simultaneous operation of the first and second HF generation units, said inverter controller being configured to receive as an input signal first and second currents, wherein according to the invention the generator further comprises a working currents calculation device having an observer unit configured for an indirect determination of first and second working currents based on measured values obtained by a dual measurement of the first and second total current outputted at the first and the second output, wherein the working currents calculation device is communicatively connected to the inverter controller and is configured to provide said first and second working currents to the inverter controller to be employed as said input signal.

In the following, some expressions that are used within the context of the invention are explained:

In the field of electrosurgical generators, "high frequency" refers to frequencies typically in the range between 200 kHz and 4000 kHz which is also being known as a radiofrequency (RF).

"High voltage" typically refers to voltages up to 10 kV, preferably up to 4000 V, further preferably at least 10 V.

In the context of the present invention, an observer unit is understood to be a device designed to determine the state of a system from measurements of its outputs. It is configured to determine a variable or state that is not, or at least not directly, accessible by using parameter and other measured variable outputted by the system. Various such observer units are known to the person skilled in the art of control systems.

The inverter unit is a device to provide the actual high-frequency alternating voltage output for the surgical instrument to be connected to the output socket. This term is rather broad and comprises inverter technology as well as converters and amplifiers.

A cross-current is a portion of the total current emitted by an output that flows to the other output in an activated state of the electrosurgical generator. A working current is the other portion of the total current, this working current actually flowing from the output via the electrosurgical instrument to the tissue to be worked on (and back to the same output in case of bipolar configuration or to a separate neutral electrode in case of monopolar configuration). The working current and the cross-current form the (total) current of the respective output. The (total) currents are measured by sensors which are provided at the outputs and/or the output lines. Accordingly, the first and second working currents are different from the first and second (total) current at the respective first and second output. The first and second working currents is a portion of said respective first and second (total) current, namely that portion which flows - in the activated state - from the respective first and second electrosurgical instrument (19, 19') into the tissue to be worked on.

The underlying problem is that the working current which is actually delivered from the electrosurgical instruments to the tissue to be worked on cannot be measured directly. This is largely due to presence of a cross-current between the dual outputs.

The invention has realized that an improved control of the outputs and the electrosurgical instruments supplied by them can be achieved by employing the working current (or resulting power) as a control signal, although said working current cannot be directly measured. While this appears to be paradoxical at first sight, the invention has realized that the difficulties as experienced with the known concepts can be overcome thereby.

The core of the invention is to employ a working current which is calculated instead of being measured - since no direct measurement is possible -, and to create an observer unit configured for an indirect determination of the working current based on measured values, in particular the (total) current output - which is directly measurable - of either output. These working currents for both outputs as determined thereby are then employed as input signals of the inverter controller.

The invention draws on the fact that owing to the first and second HF generation unit a voltage having a first frequency is applied at the first output and a voltage having a different, second frequency is applied at the second output, resulting in a beat frequency and a cross-current. As soon as a cross-current occur, the measured current at either output, however, will be a combination of currents of both frequencies. The invention has realized that this can not only be used to detect presence of cross-currents, but that the dual frequency characteristic can also be employed to determine the portion of the current delivered by the output that is an actual working current.

By virtue of this, the invention allows for a direct determination of the individual working currents for both outputs. This allows for a much-improved control of the energy delivered by the respective electrosurgical instruments. Further, the uncertainties as encountered in the prior art by a mere estimation can be effectively avoided. Quality of control can be increased as well as operational safety of the electrosurgical generator, and risks for the patient stemming from undetected or falsely estimated over-currents and/or cross-currents can be effectively reduced. Preferably, the observer unit comprises a Fourier series approximation device and an automatic coefficient determining device, said Fourier series approximation device being configured for actual calculating an approximation by dual Fourier series, one for the first working current and one for the second working current, and said automatic coefficient determining device being configured to calculate coefficients of said dual Fourier series approximation based on the measured values of the dual current outputs and to communicate said coefficients to the Fourier series approximation device. Quality of the approximation can be determined as it is known to the person skilled in the art by choosing to which order the Fourier series shall be made. Higher order provide more precise results, however this is often not necessary. In a preferred embodiment approximation to the first order, i.e. with respect to the fundamental frequency, will provide an optimum between sufficiently precise results and required calculation efforts / processing power.

The automatic coefficient determining device will be explained first using a simplified Fourier series being an approximation to the first order (fundamental frequency). It uses K measured samples of the measurable (total) current output of the first output $I_1$ to determine an offset coefficient for a Fourier series around the first frequency f1

$$a_{1,0}(k) = \frac{2}{K} \sum_{k-K}^{k} I_1(k)$$

as well as two first order coefficients:

$$a_{1,1}(k) = \frac{2}{K} \sum_{k-K}^{k} I_1(k) \cdot \cos(2 \cdot \pi \cdot f_1 \cdot k \cdot \Delta)$$

$$b_{1,1}(k) = \frac{2}{K} \sum_{k-K}^{k} I_1(k) \cdot \sin(2 \cdot \pi \cdot f_1 \cdot k \cdot \Delta)$$

using a $\Delta$ as sampling time.

Likewise, based on the measurable (total) current output of the second output $I_2$ a determination is made for an offset coefficient

$$a_{2,0}(k) = \frac{2}{K} \sum_{k-K}^{k} I_2(k)$$

as well as for the two first order coefficients:

$$a_{2,1} = \frac{2}{K} \sum_{k-K}^{k} I_2(k) \cdot \cos(2 \cdot \pi \cdot f_1 \cdot k \cdot \Delta)$$

$$b_{2,1} = \frac{2}{K} \sum_{k-K}^{k} I_2(k) \cdot \sin(2 \cdot \pi \cdot f_1 \cdot k \cdot \Delta)$$

The same is to be performed for a second Fourier series around the second frequency f2, thereby yielding a set of another six coefficients. These coefficients as determined by the automatic coefficient determining device are provided to the Fourier series approximation device of the observer unit.

In a system with two monopolar electrosurgical instruments, determination of the actual working currents flowing through the electrosurgical instrument connected to the first output, current $I_{w1}$, as well as flowing through the electrosurgical instrument connected to the second output, current $I_{w2}$, is implemented in the observer unit as exemplified below, wherein I(k) denotes a single measurement value:

$$I_{w1}(k) = \frac{a_{1,0,f_1} + a_{2,0,f_1}}{2} + (a_{1,1,f_1} + a_{2,1,f_1}) \cdot cos\,(2 \cdot \pi \cdot f_1 \cdot k \cdot \Delta) + (b_{1,1,f_1} + b_{2,1,f_1}) \cdot sin\,(2 \cdot \pi \cdot f_1 \cdot k \cdot \Delta)$$

$$I_{w2}(k) = \frac{a_{1,0,f_2} + a_{2,0,f_2}}{2} + (a_{1,1,f_2} + a_{2,1,f_2}) \cdot \cos(2 \cdot \pi \cdot f_2 \cdot k \cdot \Delta) + (b_{1,1,f_2} + b_{2,1,f_2}) \cdot \sin(2 \cdot \pi \cdot f_2 \cdot k \cdot \Delta)$$

The cross-current $I_{cross}$ can be easily determined by subtracting the respective measured total current $I_1$ (or $I_2$) from the working current $I_{w1}$ (or $I_{w2}$) from according to Kirchhoff's current law. The corresponding voltage is $V_{cross}$.

The respective resistances R of the impedances Z are calculated according to, wherein $\cos(\varphi_{u,i})$ - abbreviated as $\cos(\varphi)$ - denotes a power factor as determined by a phase shift $\varphi$ between voltage and current:

$$R_{w1} = \frac{Z_{w1}}{cos(\varphi_{u,i})} = \frac{V_{1,rms}}{I_{w1,rms} \cdot cos(\varphi_{u,i})}$$

$$R_{w2} = \frac{Z_{w2}}{cos(\varphi_{u,i})} = \frac{V_{2,rms}}{I_{w2,rms} \cdot cos(\varphi_{u,i})}$$

$$R_{cross} = \frac{Z_{cross}}{cos(\varphi_{u,i})} = \frac{V_{cross,rms}^2}{I_{cross,rms} \cdot cos(\varphi_{u,i})} = \frac{\sqrt{V_{1,rms}^2 + V_{2,rms}^2}}{I_{cross,rms} \cdot cos(\varphi_{u,i})}$$

Based on the currents, power signals delivered by either output can be determined according to

$$P_1 = V_1 \cdot I_1 \cdot cos(\varphi) + V_{cross} \cdot I_{cross} \cdot cos(\varphi) = V_1 \cdot I_1 \cdot cos(\varphi) + (V_2 - V_1) \cdot I_{cross} \cdot cos(\varphi)$$

$$P_2 = V_2 \cdot I_2 \cdot cos(\varphi) + V_{cross} \cdot I_{cross} \cdot cos(\varphi) = V_2 \cdot I_2 \cdot cos(\varphi) + (V_2 - V_1) \cdot I_{cross} \cdot cos(\varphi)$$

As input and output signals of the control, root mean squared (RMS) values of each parameter are measured with the sampling time equal to the period time of a sliding window. Due to the impedances, it is recommended to calculate the power as a function of the resistances. Following, the average power can be calculated according to

$$P_{1,avg} = \frac{V_{1,rms}^2}{R_{B1}} + \frac{V_{2,rms}^2 + V_{1,rms}^2}{R_{cross}} = \frac{R_{B1} + R_{cross}}{R_{B1} \cdot R_{cross}} \cdot V_{1,rms}^2 + \frac{1}{R_{cross}} \cdot V_{2,rms}^2$$

$$P_{2,avg} = \frac{V_{2,rms}^2}{R_{B2}} + \frac{V_{2,rms}^2 + V_{1,rms}^2}{R_{cross}} = \frac{1}{R_{cross}} \cdot V_{1,rms}^2 + \frac{R_{B2} + R_{cross}}{R_{B2} \cdot R_{cross}} \cdot V_{2,rms}^2$$

For a system with two bipolar electrosurgical instruments, the determination of the actual working currents is likewise to the determination in the monopolar system. The following applies for the cross-currents according to Kirchhoff's current law

$$I_{cross1} = -I_{cross2} = I_{w1} - I_1$$

The sum of the cross resistances $R_{cross1}$ and $R_{cross2}$ is calculated using the equation

$$R_{cross1} + R_{cross2} = \frac{\sqrt{V_{1,rms}^2 + V_{2,rms}^2}}{I_{cross1,rms} \cdot cos(\varphi_{u,i})}$$

With the assumption, that $R_{cross1}$ is equal to $R_{cross2}$, the corresponding voltages $V_{cross1}$ and $V_{cross2}$ are assumed to be related according to

$$V_{cross1} = -V_{cross2} = \frac{1}{2} \cdot (V_2 - V_1)$$

The average power at each instrument is determined according to

$$P_{1,avg} = \frac{V_{1,rms}^2}{R_{B1}} + \frac{V_{cross1,rms}^2}{R_{cross1}} = \frac{V_{1,rms}^2}{R_{B1}} + \frac{(V_{1,rms}^2 + V_{2,rms}^2)}{2 \cdot (R_{cross1} + R_{cross2})}$$

$$P_{2,avg} = \frac{V_{2,rms}^2}{R_{B2}} + \frac{V_{cross2,rms}^2}{R_{cross2}} = \frac{V_{2,rms}^2}{R_{B2}} + \frac{(V_{1,rms}^2 + V_{2,rms}^2)}{2 \cdot (R_{cross1} + R_{cross2})}$$

The power of each instrument is compared to the respective reference power and may be controlled in a power control.

For a current control, the RMS values of the output currents I1 and I2 of each instrument may be controlled manipulating the output voltages.

For a voltage control, the output voltages are compared to the voltage drop of the cross impedance(s) and the maximum is used as controlled variable manipulated through the output voltages itself.

It is an important advantage of the invention that this is accomplished at two distinct frequencies f1 and f2. Thereby, the number of available coefficients is to be doubled, enabling the observer unit to calculate not only the measurable total current output but also the non-measurable working current flowing through the electrosurgical instrument into the tissue to be worked on. By virtue of this, a precision and granularity of the determination of the currents can be realized that was not achievable before.

As it can be readily appreciated, further electrical parameters like voltages, including a cross voltage, and/or other currents, preferably a cross current, can be determined in consideration of said first and second working currents. Root mean values (RMS) for the respective currents, voltage and powers can be determined as well.

Advantageously, the observer unit further comprises a phase angle determination module configured to determine a phase angle between voltage and the first and/or second current of the first and second output, respectively. This allows for a proper consideration of the phase shift between voltage and current at either output. This is important for an assessment of active power and therefore energy transferred to the tissue by the electrosurgical instruments. Further, determination of root mean square values is facilitated by the phase angle determination module.

For efficient calculation of the Fourier transformation, the observer unit advantageously comprises a sliding window device for data processing of the measured values. Advantageously, said sliding window features a variable length, the variable length being preferably determined dependent on the first frequency, the second frequency and a frequency difference between the first and second frequency. This allows for proper fitment of the length of the sliding window to the frequencies used. For computational efficiency, preferably a fast (FFT) or discrete (DFT) Fourier transformation may be employed, however this is not a must.

Preferably, the sliding window device comprises a common denominator calculation unit and is preferably configured to use an inverse of a common denominator for the length of the sliding window. Thereby the sliding window can be fitted to the frequencies, thereby reducing unwanted spectral leakage. It is particularly advantageous to use the inverse of the greatest (or second greatest) common denominator for the length of the sliding window. This results in a short window providing an optimum with respect to frequency resolution and avoidance of spectral leakage.

Although the actual current flowing through the electrosurgical instrument is the more important parameter, it may also be beneficial to address the cross-current. For this end, advantageously a cross-current detector is provided, said cross-current detector being configured to determine a cross-current between the first and second output in an activated state. This allows for a more precise calculation of total power output of the electrosurgical generator at its dual output. Further, this enables proper consideration of the cross-current. The cross-current detector is further preferably co-operatively connected to a cross-current regulator configured to adjust electrical characteristics, in particular voltage, current and/or frequency, of the second HF generation unit such as to control the cross-current.

Preferably, output of the first and second HF generation unit is independently controlled by a respective first and second HF generation controller, preferably using the first and second working current, respectively, and optionally also the cross current as an input variable (and/or the respective impedances). Using independent controllers allows for the most specific response to varying impedances at the electrosurgical instruments, in particular due to different properties of tissue to which the respective electrosurgical instrument is applied. The load impedance of the tissue treated is an important parameter and can be readily calculated by dividing the measured voltage by the working current as determined by the observer unit. It is advantageous if the first and second generation controller are enabled to applying various control laws, preferably "Current control", and/or "Power control", optionally also "Voltage control". It is particularly preferred if the various control laws are to be employed selectively, in particular dependent on load and/or power of the respective electrosurgical instrument. It is particularly preferred if more than one control law is processed, and the outputs of each are compared and one is selected. For example an error between actual and reference value is to be determined for each, and the control law with the smallest error is used by a selection device to determine the setpoint for further control. - "Current control" aims to maintain a current and consequently the tissue effect achieved by the electrosurgical instrument. It is particularly useful for rather low load impedances. "Power control" enables precise control of the energy delivered to the electrosurgical instrument per time. It takes into account the calculated values for the working current as well as for the cross-current, as indicated in the power equations for $P_1$ and $P_2$ shown above. It is particularly useful for medium load impedance at the electrosurgical instrument. - For rather high load impedances at the electrosurgical instrument, a "voltage control" is provided, e.g. to ensure that voltages as output and applied are within safe limits for the electrosurgical instrument and the patient. The various control laws may be employed individually or parallelly, e.g. current control with an underlying voltage control. - Further preferably a decoupling controller is provided being configured for independent control of first and second HF generation unit, said decoupling controller interacting with the observer unit. Thereby, by virtue of the observer unit and in particular the calculated working currents a decoupling of control for the electrosurgical instruments at the two outputs can be achieved. Thereby adverse interference between first and second HF generation unit can be minimized or avoided, even in consideration that both are electrically connected by the crossing current. This is a major advantage in applying proper control. It is important to note that the first and second HF generation controller are enabled to operate independently from each other, including that they are configured to apply different control laws.

However, it shall also be possible that these first and second HF generation units are formed as a combined unit that is enabled to output the first as well as the second high-frequency alternating voltage independently from each other. Thereby the first and second frequency having different voltages and frequencies can be generated by a single combined unit. To this end, the combined unit is preferably a multilevel-inverter having a plurality of inverter cells, wherein a first portion of the plurality of inverter cells is configured to generate the first high-frequency alternating voltage and a second portion of the plurality of inverter cells is configured to generate the second high-frequency alternating voltage. Thereby, the first and second frequency for dual output can be generated in a very efficient manner, and the same inverter can alternatively be used with all inverter cells in one group if only one output is to be supplied. This achieves increased versatility with minimum effort.

A multi-level inverter is an inverter unit being enabled to emit an output voltage at various levels, as opposed to providing On/Off output only with positive and/or negative polarity. Typical topologies are, but not limited to, cascaded H-bridge, neutral clamped and flying capacitor.

For practical purposes, it is beneficial if the first frequency, the second frequency and the frequency difference between the first and second frequency are selected such as their respective values are a composite number, i.e. non-prime. Being non-prime, a rather great common denominator for the first, second and different frequency can be established, allowing a rather short length of the sliding window for achieving good spectral resolution and minimizing spectral leakage.

The invention is explained in more detail below with reference to an advantageous exemplary embodiment. In the figures:

Fig. 1      shows a frontal view of an electrosurgical generator with two attached electrosurgical instruments;

Fig. 2      shows a schematic view of plural electrosurgical instruments at the surgery site;

Fig. 3      shows a schematic functional diagram of the electrosurgical generator including two monopolar output sockets;

Fig. 4      shows a detail view of an observer unit;

Fig. 5      shows an equivalent circuit diagram and currents for two outputs supplying two monopolar electrosurgical instruments;

Fig. 6      shows currents for output of two different frequencies and a resulting beat frequency;

Fig. 7      shows a power diagram with different control regions;

Fig. 8      shows an alternative equivalent functional diagram to Fig. 5;

Fig. 9      shows a functional control block diagram showing a configuration for power control;

Fig. 10     shows a schematical diagram of an observer unit comprising a Fourier series approximation device and an automatic coefficient determining device;

Fig. 11     shows a schematic functional diagram of an another embodiment of the electrosurgical generator including a combined high-frequency generation unit; and

Fig. 12     shows an equivalent circuit diagram and currents for two outputs supplying two bipolar electrosurgical instruments.

**EP 4 545 029 B1**

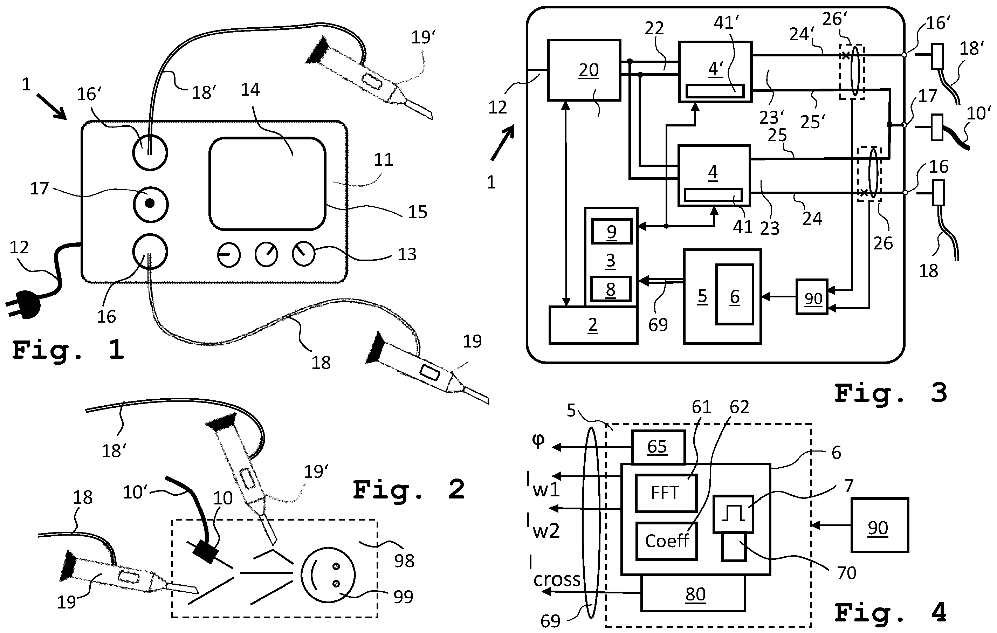

An exemplary embodiment for an electrosurgical generator according to a first embodiment of the present invention is illustrated in Fig. 1. The electrosurgical generator, identified as a whole by reference numeral 1, comprises a housing 11 having at least two output sockets 16, 16' (and optionally an additional socket 17 for a neutral electrode) for connection of electrosurgical instruments 19, 19'. A power supply cable 12 is provided with a plug for connection to an electrical power source (not shown) which may be an electricity grid, like AC mains in a building, or an off-grid source of electric energy, like a 12 Volt or 24 Volt battery in a vehicle or mobile hospital. Further, a user interface 14 is provided comprising a display 15 which may be a touchscreen, or knobs 13 may be present for inputs by a user.

Said electrosurgical instrument 19 comprises a cable 18 which is to be plugged-in into the output socket 16 in order to supply high-frequency alternating voltage for operation of the electrosurgical instrument 19. This applies likewise with respect to the other electrosurgical instrument 19' to be connected by its cable 18' to the output socket 16'.

The two electrosurgical instruments 19, 19' to be supplied by the electrosurgical generator 1 are shown in Fig. 2 at a surgery site. A surgery table 98 is provided which is configured for accommodating a patient 99 to be treated. In the depicted embodiment, both electrosurgical instruments 19, 19' are to be used on the same patient 99 at different locations. In the depicted embodiment, both electrosurgical instruments 19, 19' are of the monopolar type. This necessitates a neutral patch electrode 10 that is attached to the surgery table 98 or, preferably, to the patient 99 creating a rather large contact area. The neutral patch electrode 10 is connected via a cable 10' to the additional socket 17 of the electrosurgical generator 1, thereby providing a return path and closing the circuit for the HF energy as provided by the electrosurgical generator 1 to the electrosurgical instruments 19, 19'.

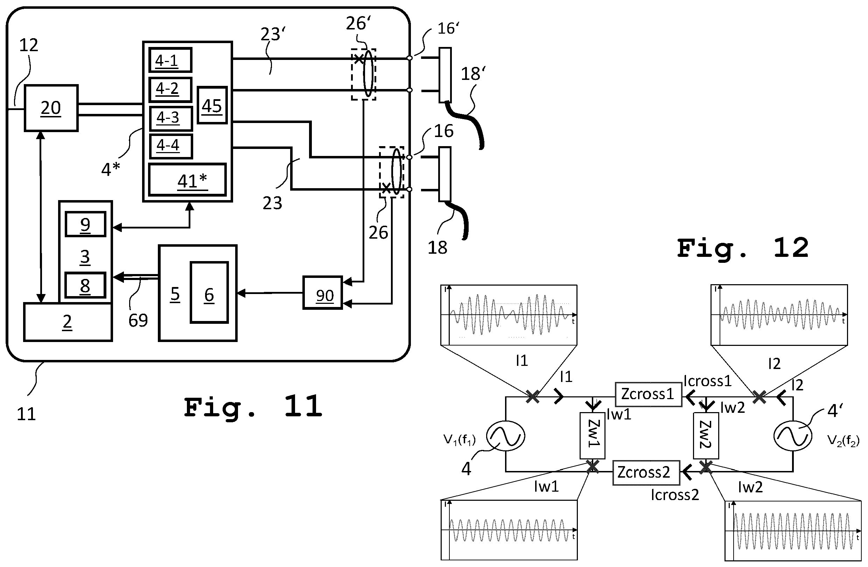

A schematic functional diagram of the electrosurgical generator is shown in Fig. 3. Electric power is delivered by the power supply cable 12 to a power supply unit 20 of the electrosurgical generator 1. The power supply unit provides electrical power, in the depicted embodiment DC power, to the various devices, units and modules of the electrosurgical generator 1. In particular, it provides electrical power via a DC bus 22 to a first HF generation unit 4 configured for generating a first high-frequency alternating voltage, said high-frequency voltage being fed via a first output line 23 and a first sensor assembly 26 for voltage and current measurement to a first output socket 16 (also termed output 16 for brevity) for supplying the plugged-in electrosurgical instrument 19. The output line 24 comprises an active electrode (AE) line 24 and a neutral electrode (NE) line 25. The high-voltage is usually in a range of a few kilovolts, but may have amplitudes in a range of several 10 Volt up to 4000 Volt. Further, a second HF generation unit 4' is provided which is also supplied from the power supply 20 via the DC Bus 22 and is provided with a second output line 23' for outputting voltage in a likewise fashion, said second output line 23' comprising an active electrode line 24' and a neutral electrode line 25', and a second sensor assembly 26' for voltage and current measurement at a second output socket 16'. In the depicted embodiment a monopolar configuration is shown, wherein the output sockets 16, 16' are configured as unipolar outputs for connection of a monopolar instrument 19, 19', and further having the neutral electrode (NE) lines 25, 25' being connected to the additional neutral output socket 17 to which the neutral patch electrode 10 is connected. For bipolar outputs a second embodiment is provided as shown in Fig. 11 and 12.

Operation of the first and second HF generation units 4, 4' is governed by a central control unit 2 which in turn is connected with the user interface 14 such that the user can issue directions and commands for operation of the electrosurgical generator 1. The central control unit 2 generates corresponding control signals and governs the relevant devices, units and modules of the electrosurgical generator 1 according to these instructions and commands. In particular, the central control unit 2 comprises a common inverter controller 3 which provides command signals to a first and second HF generation controller 41, 41', either one in turn controlling its respective first and second HF generation unit 4. The first high-frequency voltage as generated by the first HF generation unit 4 is conveyed via the respective output line 23 and the sensor assembly 26 to the output socket 16. Likewise, the second high-frequency voltage as generated by the second HF generation unit 4' is conveyed via the respective output line 23' and the sensor assembly 26' to the output socket 16'.

The first and second HF generation unit 4, 4' generate the first and second HF voltage, respectively, with a voltage and a frequency as prescribed by the respective first and second HF generation controller 41, 41'. Voltage and frequency of the first and second HF generation unit 4, 4' can be controlled and generated independently from each other. However, the resulting current which is delivered from the first and second HF generation unit 4, 4' via its respective output socket 16, 16' is determined by the impedance of the respective electrosurgical instrument 19, 19' and the tissue contacted by either instrument. The total current $I_1$ flowing through the first output socket 16 is determined by the first sensor assembly 26 which also determines the respective actual voltage; likewise, the total current $I_2$ flowing through the second output socket 16' is determined by the second sensor assembly 26' which also determines the respective actual voltage. Resulting measuring signals are applied at a signal conditioning unit 90, which may comprise an analog to digital converter (ADC), and further provides feed-back to the central control unit 2 via a working current calculation device 5.

However, the total current $I_1$ as determined by the respective sensor assembly 26, 26' is not identical to the current flowing through the tissue to which the electrosurgical instrument is applied ("working current").

As depicted in Fig. 2, the electrosurgical instruments 19, 19' are usually applied at different locations of the patient's body. These different locations can be rather close to each other, particularly in case of assisted surgeries, or can be rather far from each other, particularly in case of co-surgeries. In either case, the total current as being delivered by the output 16 of the electrosurgical instrument 19 will not only take the desired path through the tissue to be treated toward the neutral electrode 10, but will also flow to the other electrosurgical instrument 19' as a so called cross-current. The same applies likewise for the current delivered by the output 16' of the electrosurgical instrument 19' with respect to a cross-current toward the electrosurgical instrument 19.

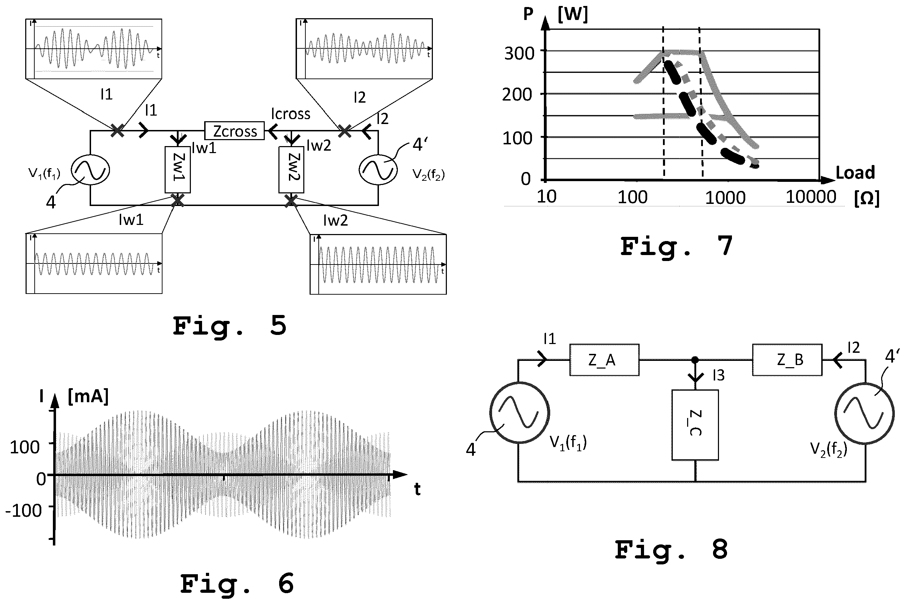

This is shown in Fig. 5 showing an equivalent circuit diagram for the case of two monopolar electrosurgical instruments 19, 19' having a shared neutral electrode 10. In said equivalent circuit diagram, at the very left as well as at the very right are shown two voltage sources which symbolize the first high-frequency generation unit 4 emitting and alternating voltage V1 having a first frequency f1 and the second high frequency generation unit 4' emitting and alternating voltage V2 having a first frequency f2. As a consequence of the high-frequency generation units 4, 4' emitting different frequencies, a beat frequency occurs.

This is also shown in Fig. 6, wherein the first high-frequency generation unit produces a voltage V1 at the first frequency f1 and the second high-frequency generation unit 4' produces a voltage to at the second frequency f2. The currents generated by these voltages are shown by the waves having a large amplitude (both frequencies are rather similar, they can hardly be distinguished in the figure as both are having the same amplitude). The resulting beat frequency is depicted by the wave having a lesser amplitude. This beat frequency occurs due to a cross-coupling induced by a cross impedance of the patient's body 99, as will be illustrated next.

The total current $I_1$ as supplied by the output 16 and flowing via the cable 18 to the electrosurgical instrument 19 is shown in the upper left-hand corner in the sub-diagram, and likewise the total current $I_2$ as supplied by the output 16' and flowing via the cable 18' to the other electrosurgical instrument 19' is showing in the sub-diagram positioned in the upper righthand corner. As it can be readily appreciated, the current flow is affected by the beat frequency.

Either current as supplied by either high-frequency generation unit 4, 4' splits into one portion $I_{cross}$, flowing through a cross impedance formed by the patient body 99 towards the respective other high-frequency generation unit 4', 4, and one other portion $I_{w1}$ or $I_{w2}$, flowing through the respective tissue impedance $Z_{w1}$, $Z_{w2}$ towards the shared neutral patch electrode 10 (and ultimately back to the high-frequency generation units 4, 4'). It is the (working) currents $I_{w1}$ and $I_{w2}$ flowing through the tissue impedance that need to be properly controlled for effective treatment. However, these working currents cannot be directly measured due to the presence of the cross-current $I_{cross}$. Directly measurable is only either total current $I_1$ and $I_2$ as supplied by the respective high-frequency generation unit 4, 4'.

For proper control of the power and energy delivered by the respective electrosurgical instrument 19, 19' to the tissue to be treated, the respective working currents $I_{w1}$ or $I_{w2}$, need to be known. However, as already stated, they cannot be measured directly. In order to be enabled for proper control, the invention provides for a working currents calculation device 5 which is configured to determine $I_{w1}$ or $I_{w2}$ indirectly by means of an observer unit 6 which receives quantities that can be measured as input values. By the same token, the cross current $I_{cross}$ can be determined and can subsequently be utilized for current and power control.

As shown in Fig. 2 and 4, the measured quantities for total currents $I_1$ and $I_2$, as well as measured voltages, of the respective output 16, 16' are sensed by the respective sensor assembly 26, 26' and the measured values are provided to a signal conditioning unit 90, which in particular comprises an analog to digital converter (ADC), and is subsequently provided to the observer unit 6. The sample data is processed by a sliding window device 7. The length of the sliding window employed is variable and depends on the first frequency f1, namely the frequency of the voltage generated by the first high-frequency generation unit 4, and on the second, distinct frequency f2, namely the frequency of the voltages generated by the second high-frequency generation unit 4', as well as on a frequency difference $\Delta f$ between said first and said second frequency. Based on these values, their common denominators are calculated by a common denominator device 70. Employing the common denominators as determined thereby, the length of the sliding window is defined by the inverse of the, preferably greatest, common denominator. An illustrative example (with exaggerated numbers for better illustration) has a first frequency f1 of 300 kHz, a second frequency of 400 kHz leading to a $\Delta f$ of 100 kHz. The greatest common denominator of these numbers is 100k, leading to a window length of 10 $\mu s$ ($10^{-5}$ s). At an assumed sampling frequency of 150M/s this results in 150M/s * 10 $\mu s$ = 1500 samples for the window length. Thereby a rather short window length can be achieved which realizes a high spectral resolution with minimum spectral leakage. The resulting windowed data is provided to a Fourier series approximation device 61 and to an automatic coefficient determining device 62 which is configured to calculate coefficients for said Fourier series approximation device 61.

The automatic determination of the coefficients for the Fourier series as well as a Fourier transformation will be accomplished by the Fourier series approximation device 61 and the automatic coefficient determining device 62 as shown in the following:

Based on the measured total current $I_1$ as supplied by the output 16 to the first electrosurgical instrument 19 the Fourier coefficients for a first order Fourier series with offset will be determined in order to obtain the working current $I_{w1}$:

$$a_{1,0}(k) = \frac{2}{K} \sum_{k-K}^{k} I_1(k)$$

$$a_{1,1}(k) = \frac{2}{K} \sum_{k-K}^{k} I_1(k) \cdot \cos(2 \cdot \pi \cdot f_1 \cdot k \cdot \Delta)$$

$$b_{1,1}(k) = \frac{2}{K} \sum_{k-K}^{k} I_1(k) \cdot \sin(2 \cdot \pi \cdot f_1 \cdot k \cdot \Delta)$$

This is performed by the automatic coefficient determining device 62. Likewise, the coefficients for a corresponding Fourier series relating to the working current $I_{w2}$ of the second electrosurgical instrument 19' are determined by the automatic coefficient determining device 62:

$$a_{2,0}(k) = \frac{2}{K} \sum_{k-K}^{k} I_2(k)$$

$$a_{2,1} = \frac{2}{K} \sum_{k-K}^{k} I_2(k) \cdot \cos(2 \cdot \pi \cdot f_1 \cdot k \cdot \Delta)$$

$$b_{2,1} = \frac{2}{K} \sum_{k-K}^{k} I_2(k) \cdot \sin(2 \cdot \pi \cdot f_1 \cdot k \cdot \Delta)$$

These coefficients are supplied from the automatic coefficient determining device 62 to the Fourier approximation device 61, which in turn calculates the working currents using a first order Fourier series:

$$I_{w1}(k) = \frac{a_{1,0,f_1} + a_{2,0,f_1}}{2} + (a_{1,1,f_1} + a_{2,1,f_1}) \cdot cos\,(2 \cdot \pi \cdot f_1 \cdot k \cdot \Delta) + (b_{1,1,f_1} + b_{2,1,f_1}) \cdot sin\,(2 \cdot \pi \cdot f_1 \cdot k \cdot \Delta)$$

$$I_{w2}(k) = \frac{a_{1,0,f_2} + a_{2,0,f_2}}{2} + \left(a_{1,1,f_2} + a_{2,1,f_2}\right) \cdot \cos(2 \cdot \pi \cdot f_2 \cdot k \cdot \Delta) + (b_{1,1,f_2} + b_{2,1,f_2}) \cdot \sin\,(2 \cdot \pi \cdot f_2 \cdot k \cdot \Delta)$$

Thereby, by means of the Fourier series approximation device 61 with the automatically determined coefficients values for the - not directly measurable - currents $I_{w1}$ and $I_{w2}$ can be obtained. They are conveyed to the central control unit 2, in particular its common inverter controller 3 by means of a multi-parameter signal 69 which preferably also comprises measurement signals for the voltage measured at either output 16, 16'. This allows calculation of power.

Further, the observer unit 6 is provided with a phase angle determination module 65. It is configured to determine a phase angle φ between voltage and the first and/or second current $I_1$, $I_2$ of the first and second output 16, 16', respectively. This allows for determining an actual phase difference φ at either output 16, 16', thereby enabling a proper consideration of the phase shift between voltage and current supplied to the electrosurgical instrument 19, 19' at the respective first and second output 16, 16'. This is essential for an assessment of active power and therefore energy transferred to the tissue by the electrosurgical instruments. Further, root mean square values can be determined using the phase angle φ as provided by said phase angle determination module 65.

The common inverter controller 3 is further provided with a cross-current regulator 8. It is communicatively connected to a cross-current detector 80 to which data of the observer unit 6, in particular of its Fourier series approximation device 61, is supplied. Thereby, presence of cross-current $I_{cross}$ can be detected, and corresponding signals can be supplied to the cross-current regulator 8. The cross-current regulator 8 cooperates with the common inverter controller 3 to modify driving of the high-frequency generation units 4, 4', in particular their respective HF generation controller 41, 41', in order to control their output in consideration of the cross-current. This can e.g. realized by a current control since effect on the tissue is determined by the total current which is the sum of working current and cross-current. Similarly, a power control can take advantage of the cross-current in that the total power acting on the tissue is the sum of the power delivered to the Body Impedance Zw1 and Zw2, respectively, and to the Crossbody Impedance Zcross. Further, with respect to voltage control the tissue is affected by the maximum of the voltage drop across the Body Impedance Zw1 or Zw2, respectively and Zcross.

Employing the hereby determined working currents $I_{w1}$ and $I_{w2}$, various control laws are implemented in the common inverter controller 3 and the first and second HF generation controller 41, 41'. Owing to the working current as determined by the observer unit 6, the actual load impedance for either electrosurgical instrument 16 can be readily determined by taking the respective voltage as measured V1 for the electrosurgical instrument 16 and dividing it by the determined working current $I_{w1}$ (voltage V2 divided by working current $I_{w2}$ in case of the second electrosurgical instrument 19'). In Fig. 7 a power diagram is shown with different control regions. Three embodiments are depicted wherein up to three control laws are implemented. Preferably, the various control laws are to be employed sequentially, in particular dependent on load and/or power of the respective electrosurgical instrument 19, 19'. It is to be noted that owing to the independent first and second HF generation controllers 41, 41' both high-frequency generation units 4, 4' can be operated with different control strategies and control laws.

In a first embodiment shown by the upper solid line, for a rather low load impedance, in depicted embodiment e.g. being less than about 200 Ω, "Current control" may be beneficial as it strives to achieve a defined working current. For medium load impedance, in the depicted embodiment e.g. in the range between about 200 Ω and about 500 Ω, "Power Control" may be beneficial in order to maintain a defined power delivered by the working current. For higher load impedances being greater than about 500 Ω (up to about 2000 Ω where the control law ends), preferably a "Voltage Control" is to be used in order to avoid excess voltages. This sequence of control law is applicable if the electrosurgical generator is allowed to operate at full power. If power is restricted (e.g. to "Half Power") then the lower solid line is applicable, having "Power Control" for any load impedance below about 1000 Ω, above that switching to "Voltage Control".

In a second embodiment shown by the long-dashed line, e.g. used for a different generator mode for a different surgical application, "Current Control" is performed for load impedances below about 200 Ω, above this threshold switching to "Voltage Control".

In a third embodiment shown by the dotted line, e.g. used for a yet another generator mode for a further different surgical application, "Current Control" is performed for load impedances below about 200 Ω, then briefly switching to a "Power Control" with power linearly decreasing for increasing load impedance, and thereafter about 300 Ω switching to "Voltage Control" for any higher load impedance.

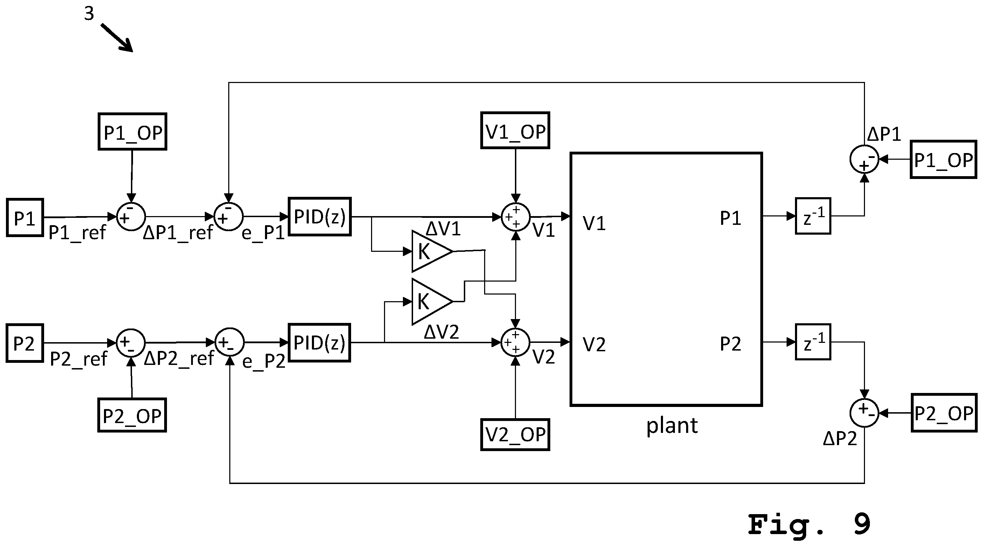

Fig. 9 shows a functional control block diagram of a configuration for power control. As already mentioned above, by "power control" the control system strives to maintain a set power. Accordingly, PID-controllers are provided which vary voltage delivered to the respective electrosurgical instrument 19, 19' in order maintain the set power. The power actually delivered is determined by the voltage and the respective working current, as determined by the observer unit 6 described above and fed back to an input of the PID controller. The PID controller may parameterized by known standard procedures, e.g. those of Ziegler-Nichols or by modern computer-assisted tools, like the Control Design Toolbox of Matlab/Simulink®.

In Fig. 8 an alternative equivalent functional diagram to that shown in Fig. 5 is depicted. It shows an additional effect, namely the emergence of a common path to the shared neutral electrode. This configuration shows the impedances arranged in a "T" manner, as opposed to the diagram in Fig. 5 which shows the impedances in a "π" (pi) manner. Although both models are usable to describe different effects they can be converted into each other utilizing the relations:

$$Z\_A = \frac{Z_{w1} \cdot Z_{cross}}{Z_{w1} + Z_{cross} + Z_{w2}}$$

$$Z\_B = \frac{Z_{w2} \cdot Z_{cross}}{Z_{w1} + Z_{cross} + Z_{w2}}$$

$$Z\_C = \frac{Z_{w1} \cdot Z_{w2}}{Z_{w1} + Z_{cross} + Z_{w2}}$$

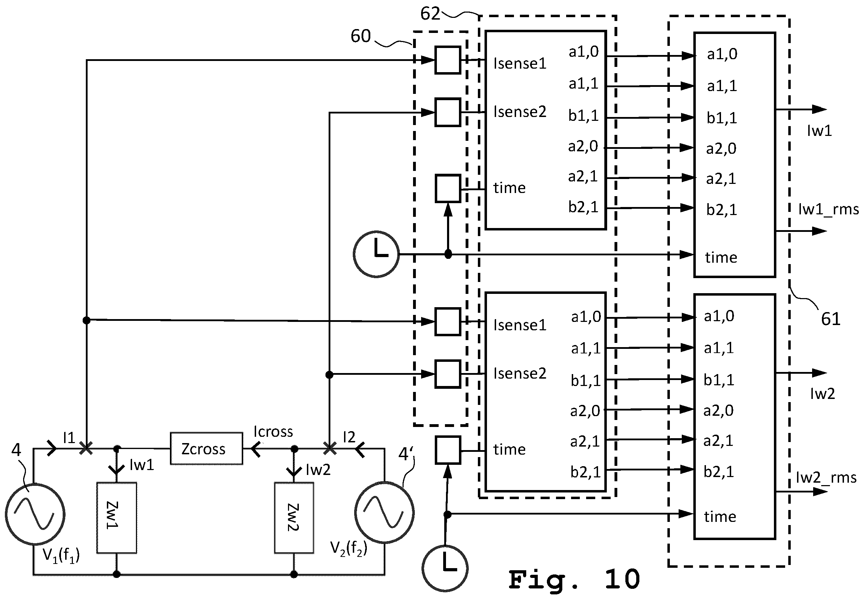

A schematical diagram of an observer unit comprising the Fourier series approximation device and the automatic coefficient determining device is shown in Fig. 10. Accordingly, the measured total currents $I_1$ and $I_2$ are provided via

buffers 60 to inputs of the automatic coefficient determining device 62 which also receives a clock signal for the sampling frequency. Based on the formulas provided above, the automatic coefficient determining device 62 is configured to determine the coefficients $a_{1,0} a_{1,1} b_{1,1}$ and $a_{2,0} a_{2,1} b_{2,1}$ for a first order Fourier approximation with respect to both working currents $I_{W1}$ and $I_{W2}$. Values of these coefficients are supplied to the Fourier series approximation device 61 which is configured to determine the working currents Iw1, Iw2 by means of a first order Fourier approximation, as already mentioned above.

In Fig. 11 a schematic functional diagram of another embodiment of the electrosurgical generator is shown. This embodiment features a bipolar configuration for bipolar electrosurgical instruments, wherein the output sockets 16, 16' are configured as bipolar outputs for connection of a bipolar instrument 19, 19', each output socket 16, 16' being supplied by output lines 23, 23' having its own pair of active electrode (AE) and neutral electrode (NE) lines.

The respective equivalent circuit diagram for such a configuration with two bipolar electrosurgical instruments is shown in Fig. 12. Similar to the configuration shown in Fig. 5, there exists a cross-resistance Zcross1 between the active electrodes of the respective electrosurgical instruments, through which a cross-current Icross1 flows. However, different from Fig. 5 no shared neutral electrode is present since each of the bipolar electrosurgical instruments features its own neutral electrode. As a consequence, however, an additional current path may appear between the respective neutral electrodes forming a cross-resistance Zcross2, resulting in an additional cross-current Icross2.

As a further difference to the embodiment of Fig. 3, the embodiment of Fig. 11 features a combined high-frequency generation unit 4* which replaces the two separate high-frequency generation units 4, 4'. It is embodied as a multi-level inverter having a plurality of inverter cells 4-1, 4-2, 4-3, 4-4. The inverter cells 4-1, 4-2, 4-3, 4-4 are being connected in a cascaded manner in a first state and are being split in two groups (e.g. two inverter cells each, but other distributions between the groups are possible), each group being connected to one of the outputs 16, 16'. Thereby, the inverter cells of either group generate the high-frequency alternating voltage for its respective output 16, 16'. The HF generation for both groups is controlled by the combined HF generation controller 4* which thus performs the functionality of the first and second HF generation controller 41, 41'. However, providing the combined high-frequency generation unit 4* is independent of the bipolar configuration. The combined high-frequency generation unit 4* can likewise be configured for monopolar configuration of the output sockets as shown in Fig. 3, and the bipolar configuration can also be applied to the dual separate high-frequency generation units 4, 4' as shown in Fig. 3. Further, a mixed arrangement for one monopolar and one bipolar output socket and respective electrosurgical instruments is possible.

As an additional benefit, this combined high-frequency generation unit 4* further allows (in its first state) to remove the grouping and to configure all of the inverter cells 4-1, 4-2, 4-3, 4-4 such as to be in one cascade, thereby supplying only one output 16 with doubled voltage range and power. This switching between the first and second state is governed by a cell configuration governor 45 which is controlled by the central control unit 2.

## Claims

1. Electrosurgical generator configured to output a high-frequency alternating voltage to electrosurgical instruments, comprising

   a first HF generation unit (4) supplying a first high-frequency alternating voltage having a first frequency to a first output (16), a second HF generation unit (4') supplying a second high-frequency alternating voltage having a second frequency which is different from the first frequency to a second output (16'),
   the first and second output being configured for connection of a first and second electrosurgical instrument (19, 19') such that in an activated state a first total current flows to the first electrosurgical instrument and simultaneously a second total current flows to the second electrosurgical instrument, and
   a central control unit (2) configured to control operation of the electrosurgical generator (1) including an inverter controller (3) for operation of the first and second HF generation units (4, 4'), said inverter controller (3) being configured to receive as an input signal first and second currents,
   **characterized in that**
   the electrosurgical generator further comprises a working currents calculation device (5) having an observer unit (6) configured for an indirect determination of first and second working currents based on measured values obtained by a dual measurement of the first and second total current as outputted at the first and the second output (16, 16'),
   the working currents calculation device (5) being communicatively connected to the inverter controller (3) and is configured to provide said first and second working currents to the inverter controller (3) to be employed as said input signal.

2. Electrosurgical generator of claim 1, **characterized in that** the observer unit (6) is provided with a Fourier series approximation device (61) for actual calculating a dual Fourier series approximation, one for the first working current and one for the second working current, and

further comprises an automatic coefficient determining device (62) being configured to calculate coefficients of the Fourier series approximation based on the measured values of the total first and second current as outputted.

3. Electrosurgical generator according to the preceding claim, **characterized in that** the working currents calculation device (5) and/or the observer unit (6) is further configured to determine other currents, preferably a cross current, as well as voltages, preferably a cross voltage, in consideration of said first and second working currents, and/or root mean values (RMS) for the respective currents, voltage and powers are to be measured and calculated.

4. Electrosurgical generator according to any of the preceding claims, **characterized in that** the observer unit (6) further comprises a phase angle determination module (65) configured to determine a phase angle between voltage and the first and/or second current of the first and second output (16, 16'), respectively.

5. Electrosurgical generator according to any of the preceding claims, **characterized in that** the observer unit (6) further comprises a sliding window device (7) for data processing of the measured values, said sliding window having preferably a variable length.

6. Electrosurgical generator according to the preceding claim, **characterized in that** the variable length is determined dependent on the first frequency, the second frequency and or a frequency difference between the first and second frequency.

7. Electrosurgical generator according to the preceding claim, **characterized in that** the sliding window device (7) comprises a common denominator calculation device (70), and is preferably configured to use an inverse of the, in particular greatest, common denominator for the length of the sliding window.

8. Electrosurgical generator according to any of the preceding claims, **characterized in that** a cross-current detector (80) is provided, said detector being configured to determine a cross-current between the first and second output (16, 16') in the activated state.

9. Electrosurgical generator according to the preceding claim, **characterized in that** the cross-current detector (80) is co-operatively connected to a cross-current regulator (8) configured to adjust electrical characteristics, in particular voltage, current and/or frequency, of the second HF generation unit (4') such as to control the cross-current.

10. Electrosurgical generator according to any of the preceding claims, **characterized in that** output of the first and second HF generation unit (4, 4') is independently controlled by a respective first and second generation controller (41, 41'), preferably using the first and second working current, respectively, as an input variable, wherein further preferably a decoupling controller (9) is provided being configured for independent control of first and second HF generation unit (4, 4'), said decoupling controller interacting with the observer unit (6).

11. Electrosurgical generator according to the preceding claim, **characterized in that** the first and second generation controllers (41, 41') are enabled to applying various control laws, preferably current control, voltage control, and/or power control.

12. Electrosurgical generator according to the preceding claim, **characterized in that** the various control laws are to be employed selectively, in particular dependent on load and/or power of the respective electrosurgical instrument (19, 19').

13. Electrosurgical generator according to any of the preceding claims, **characterized in that** the first frequency, the second frequency and the frequency difference between the first and second frequency are selected such as their respective values are a composite number, i.e. non-prime.

14. Electrosurgical generator according to any of the preceding claims, **characterized in that** the first and second HF generation units (4, 4') are formed as a combined unit (4*) being enabled to output the first and second high-frequency alternating voltage at the first and second output, respectively.

15. Electrosurgical generator according to the preceding claim wherein the combined unit (4*) is a multilevel-inverter having a plurality of inverter cells (4-1, 4-2, 4-3, 4-4), wherein a first portion of the plurality of inverter cells (4-1, 4-2) is configured to generate the first high-frequency alternating voltage and a second portion of the plurality of inverter cells (4-3, 4-4) is configured to generate the second high-frequency alternating voltage.

**Patentansprüche**

1. Elektrochirurgischer Generator, der ausgebildet ist eine hochfrequente Wechselspannung an elektrochirurgische Instrumente auszugeben, umfassend

   eine erste HF-Erzeugungseinheit (4), die eine erste Hochfrequenz-Wechselspannung mit einer ersten Frequenz an einen ersten Ausgang (16) liefert, eine zweite HF-Erzeugungseinheit (4'), die eine zweite Hochfrequenz-Wechselspannung mit einer zweiten Frequenz, die sich von der ersten Frequenz unterscheidet, an einen zweiten Ausgang (16') liefert,
   wobei der erste und der zweite Ausgang zum Anschluss eines ersten und eines zweiten elektrochirurgischen Instruments (19, 19') ausgelegt sind, derart, dass in einem aktivierten Zustand ein erster Gesamtstrom zum ersten elektrochirurgischen Instrument und gleichzeitig ein zweiter Gesamtstrom zum zweiten elektrochirurgischen Instrument fließt, und
   eine zentrale Steuereinheit (2), die zur Steuerung des Betriebs des elektrochirurgischen Generators (1) ausgebildet ist und eine Wechselrichtersteuerung (3) zum Betrieb der ersten und zweiten HF-Erzeugungseinheiten (4, 4') umfasst, wobei die Wechselrichtersteuerung (3) so ausgebildet ist, dass sie einen ersten und einen zweiten Strom als Eingangssignal erhält,
   **dadurch gekennzeichnet, dass**
   der elektrochirurgische Generator ferner umfasst eine Arbeitsstromberechnungseinrichtung (5) mit einer Beobachtereinheit (6), die ausgebildet ist zur indirekten Bestimmung eines ersten und eines zweiten Arbeitsstroms auf der Grundlage von Messwerten, die durch eine duale Messung des ersten und des zweiten Gesamtstroms, wie am ersten und am zweiten Ausgang (16, 16') ausgegeben, erhalten werden,
   wobei die Arbeitsstromberechnungseinrichtung (5) kommunikativ mit der Wechselrichtersteuerung (3) verbunden ist und so konfiguriert ist, dass sie der Wechselrichtersteuerung (3) die ersten und zweiten Arbeitsströme bereitstellt zur Verwendung als das Eingangssignal.

2. Elektrochirurgischer Generator nach Anspruch 1, **dadurch gekennzeichnet, dass** die Beobachtereinheit (6) versehen ist mit einer Fourier-Reihen-Approximationseinrichtung (61), um eine duale Fourier-Reihen-Approximation zu berechnen, eine für den ersten Arbeitsstrom und eine für den zweiten Arbeitsstrom, und
   ferner eine automatische Koeffizientenbestimmungseinrichtung (62) umfasst, die dazu ausgebildet ist, um Koeffizienten der Fourier-Reihen-Approximation zu berechnen auf Grundlage der gemessenen Werte des ersten und zweiten Gesamtstroms, wie ausgegeben.

3. Elektrochirurgischer Generator nach dem vorangehenden Anspruch, **dadurch gekennzeichnet, dass** die Arbeitsstromberechnungseinrichtung (5) und/oder die Beobachtereinheit (6) ferner dazu ausgebildet ist, dass sie andere Ströme, vorzugsweise einen Querstrom, sowie Spannungen, vorzugsweise eine Querspannung, bestimmt unter Berücksichtigung des ersten und des zweiten Arbeitsstroms,
   und/oder
   Effektivwerte (RMS) für die jeweiligen Ströme, Spannungen und Leistungen zu messen und zu berechnen sind.

4. Elektrochirurgischer Generator gemäß einem der vorangehenden Ansprüche, **dadurch gekennzeichnet, dass** die Beobachtereinheit (6) ferner ein Phasenwinkel-Bestimmungsmodul (65) umfasst, das dazu ausgebildet ist, einen Phasenwinkel zwischen der Spannung und dem ersten und/oder zweiten Strom des ersten bzw. zweiten Ausgangs (16, 16') zu bestimmen.

5. Elektrochirurgischer Generator nach einem der vorangehenden Ansprüche, **dadurch gekennzeichnet, dass** die Beobachtereinheit (6) ferner eine Gleitfenstervorrichtung (7) zur Datenverarbeitung der Messwerte umfasst, wobei das Gleitfenster vorzugsweise eine variable Länge aufweist.

6. Elektrochirurgischer Generator nach dem vorangehenden Anspruch, **dadurch gekennzeichnet, dass** die variable Länge bestimmt ist in Abhängigkeit von der ersten Frequenz, der zweiten Frequenz und/oder einer Frequenzdifferenz zwischen der ersten und der zweiten Frequenz.

7. Elektrochirurgischer Generator nach dem vorangehenden Anspruch, **dadurch gekennzeichnet, dass** die Gleitfenseleinrichtung (7) eine Einrichtung zur Berechnung des gemeinsamen Teilers (70) umfasst und vorzugsweise dazu ausgebildet ist, dass sie einen Kehrwert des, insbesondere größten, gemeinsamen Teilers für die Länge des Gleitfensters verwendet.

8. Elektrochirurgischer Generator nach einem der vorangehenden Ansprüche, **dadurch gekennzeichnet, dass** ein Querstromdetektor (80) vorgesehen ist, der dazu ausgebildet ist, dass er im aktivierten Zustand einen Querstrom zwischen dem ersten und dem zweiten Ausgang (16, 16') ermittelt.

9. Elektrochirurgischer Generator nach dem vorangehenden Anspruch, **dadurch gekennzeichnet, dass** der Querstromdetektor (80) mit einem Querstromregler (8) zusammenwirkt, der dazu ausgebildet ist, um elektrische Eigenschaften, insbesondere Spannung, Strom und/oder Frequenz, der zweiten HF-Erzeugungseinheit (4') so einzustellen, dass der Querstrom geregelt wird.

10. Elektrochirurgischer Generator nach einem der vorangehenden Ansprüche, **dadurch gekennzeichnet, dass** die Leistung der ersten und der zweiten HF-Erzeugungseinheit (4, 4') unabhängig voneinander durch eine jeweilige erste und zweite Erzeugungssteuerung (41, 41') gesteuert wird, vorzugsweise unter Verwendung des ersten bzw. des zweiten Arbeitsstroms als Eingangsgröße,
wobei ferner vorzugsweise eine Entkopplungscontroller (9) vorgesehen ist, der zur unabhängigen Steuerung der ersten und zweiten HF-Erzeugungseinheit (4, 4') ausgelegt ist, wobei der Entkopplungscontroller mit der Beobachtereinheit (6) zusammenwirkt.

11. Elektrochirurgischer Generator gemäß dem vorangehenden Anspruch, **dadurch gekennzeichnet, dass** die erste und die zweite Erzeugungssteuerung (41, 41') dazu ausgelegt sind, verschiedene Regelungsgesetze anzuwenden, vorzugsweise Stromregelung, Spannungsregelung und/oder Leistungsregelung.

12. Elektrochirurgischer Generator gemäß dem vorangehenden Anspruch, **dadurch gekennzeichnet, dass** die verschiedenen Regelungsgesetze selektiv anzuwenden sind, insbesondere in Abhängigkeit von der Last und/oder der Leistung des jeweiligen elektrochirurgischen Instruments (19, 19').

13. Elektrochirurgischer Generator nach einem der vorangehenden Ansprüche, **dadurch gekennzeichnet, dass** die erste Frequenz, die zweite Frequenz und die Frequenzdifferenz zwischen der ersten und der zweiten Frequenz so gewählt sind, dass ihre jeweiligen Werte eine zusammengesetzte Zahl, d. h. keine Primzahl, sind.

14. Elektrochirurgischer Generator nach einem der vorangehenden Ansprüche, **dadurch gekennzeichnet, dass** die erste und die zweite HF-Erzeugungseinheit (4, 4') als eine kombinierte Einheit (4*) ausgebildet sind, die befähigt ist, die erste bzw. die zweite Hochfrequenz-Wechselspannung am ersten bzw. zweiten Ausgang auszugeben.

15. Elektrochirurgischer Generator nach dem vorangehenden Anspruch, wobei die kombinierte Einheit (4*) ein Multilevel-Inverter mit einer Vielzahl von Wechselrichterzellen (4-1, 4-2, 4-3, 4-4) ist, wobei ein erster Teilsatz der mehreren Wechselrichterzellen (4-1, 4-2) so konfiguriert ist, dass er die erste Hochfrequenz-Wechselspannung erzeugt, und ein zweiter Teilsatz der mehreren Wechselrichterzellen (4-3, 4-4) so konfiguriert ist, dass er die zweite Hochfrequenz-Wechselspannung erzeugt.


**Revendications**

1. Générateur electrochirurgical configuré pour sortir une tension alternative haute fréquence vers des instruments électrochirurgicaux, comprenant une première unité de génération HF (4) fournissant une première tension alternative haute fréquence ayant une première fréquence à une première sortie (16), une seconde unité de génération HF (4') fournissant une seconde tension alternative haute fréquence ayant une seconde fréquence qui est différente de la première fréquence à une seconde sortie (16'),

la première et la seconde sortie sont configurées pour connecter un premier et un second instrument électrochirurgical (19, 19') de telle sorte qu'à l'état activé, un premier courant total circule vers le premier instrument électrochirurgical et simultanément qu'un second courant total circule vers le second instrument électrochirurgical, et
une unité de commande centrale (2) configurée pour commander le fonctionnement du générateur électrochirurgical (1) comportant un contrôleur d'onduleur (3) pour le fonctionnement des première et seconde unités de génération HF (4, 4'), ledit contrôleur d'onduleur (3) étant configuré pour recevoir en tant que signal d'entrée des premier et second courants, **caractérisé en ce que**

le générateur électrochirurgical comprend en outre un dispositif de calcul de courants de travail (5) ayant une unité d'observation (6) configurée pour une détermination indirecte de premier et second courants de travail sur la base de valeurs mesurées obtenues par une mesure double des premier et second courants totaux tels qu'ils sont sortis au niveau des première et seconde sorties (16, 16'),

le dispositif de calcul de courants de travail (5) étant connecté en communication avec le contrôleur d'onduleur (3) et est configuré pour fournir lesdits premier et second courants de travail au contrôleur d'onduleur (3) à utiliser en tant que signal d'entrée.

2. Générateur électrochirurgical selon la revendication 1, **caractérisé en ce que** l'unité d'observation (6) est pourvue d'un dispositif d'approximation par série de Fourier (61) pour calculer effectivement une approximation par série de Fourier double, une pour le premier courant de travail et une pour le second courant de travail, et

comprend en outre un dispositif de détermination automatique de coefficient (62) étant configuré pour calculer des coefficients de l'approximation par série de Fourier sur la base des valeurs mesurées des premier et second courants totaux tels qu'ils sont sortis.

3. Générateur électrochirurgical selon la revendication précédente, **caractérisé en ce que** le dispositif de calcul de courants de travail (5) et/ou l'unité d'observation (6) sont en outre configurés pour déterminer d'autres courants, de préférence un courant croisé, ainsi que des tensions, de préférence une tension croisée, en tenant compte desdits premier et second courants de travail, et/ou

les valeurs efficaces (RMS) des courants, tensions et puissances respectifs doivent être mesurées et calculées.

4. Générateur électrochirurgical selon l'une quelconque des revendications précédentes, **caractérisé en ce que** l'unité d'observation (6) comprend en outre un module de détermination d'angle de phase (65) configuré pour déterminer un angle de phase entre la tension et le premier et/ou le second courant de la première et de la seconde sortie (16, 16'), respectivement.

5. Générateur électrochirurgical selon l'une quelconque des revendications précédentes, **caractérisé en ce que** l'unité d'observation (6) comprend en outre un dispositif à fenêtre glissante (7) pour le traitement de données des valeurs mesurées, ladite fenêtre glissante ayant de préférence une longueur variable.

6. Générateur électrochirurgical selon la revendication précédente, **caractérisé en ce que** la longueur variable est déterminée en fonction de la première fréquence, de la seconde fréquence et/ou d'une différence de fréquence entre la première et la seconde fréquence.

7. Générateur électrochirurgical selon la revendication précédente, **caractérisé en ce que** le dispositif à fenêtre glissante (7) comprend un dispositif de calcul de dénominateur commun (70), et est de préférence configuré pour utiliser un inverse du dénominateur commun, en particulier le plus grand dénominateur commun, pour la longueur de la fenêtre glissante.

8. Générateur électrochirurgical selon l'une quelconque des revendications précédentes, **caractérisé en ce qu'**un détecteur de courant croisé (80) est prévu, ledit détecteur étant configuré pour déterminer un courant croisé entre la première et la seconde sortie (16, 16') dans l'état activé.

9. Générateur électrochirurgical selon la revendication précédente, **caractérisé en ce que** le détecteur de courant croisé (80) est connecté de manière coopérante à un régulateur de courant croisé (8) configuré pour ajuster les caractéristiques électriques, en particulier la tension, le courant et/ou la fréquence, de la seconde unité de génération HF (4') de manière à commander le courant croisé.

10. Générateur électrochirurgical selon l'une quelconque des revendications précédentes, **caractérisé en ce que** la sortie de la première et de la seconde unité de génération HF (4, 4') est commandée indépendamment par un premier et un second régulateur de génération (41, 41'), de préférence en utilisant le premier et le second courant de travail, respectivement, en tant que variable d'entrée, dans lequel en outre de préférence un contrôleur de découplage (9) fourni est configuré pour commander indépendamment la première et la seconde unité de génération HF (4, 4'), ledit contrôleur de découplage interagissant avec l'unité d'observation (6).

11. Générateur électrochirurgical selon la revendication précédente, **caractérisé en ce que** les premier et second contrôleurs de génération (41, 41') sont autorisés à appliquer différentes lois de commande, de préférence une commande de courant, une commande de tension et/ou une commande de puissance.

12. Générateur électrochirurgical selon la revendication précédente, **caractérisé en ce que** les différentes lois de commande doivent être utilisées sélectivement, en particulier en fonction de la charge et/ou de la puissance de l'instrument électrochirurgical (19, 19') respectif.

13. Générateur électrochirurgical selon l'une quelconque des revendications précédentes, **caractérisé en ce que** la première fréquence, la seconde fréquence et la différence de fréquence entre la première et la seconde fréquence sont choisies de telle sorte que leurs valeurs respectives soient un nombre composite, c'est-à-dire non premier.

14. Générateur électrochirurgical selon l'une quelconque des revendications précédentes, **caractérisé en ce que** les première et seconde unités de génération HF (4, 4') sont formées en tant qu'unité combinée (4*) qui est autorisée à sortir la première et la seconde tension alternative haute fréquence au niveau de la première et de la seconde sortie, respectivement.

15. Générateur électrochirurgical selon la revendication précédente, dans lequel l'unité combinée (4*) est un onduleur multiniveau ayant une pluralité de cellules d'onduleur (4-1, 4-2, 4-3, 4-4), dans lequel une première partie de la pluralité de cellules d'onduleur (4-1, 4-2) est configurée pour générer la première tension alternative haute fréquence et une seconde partie de la pluralité de cellules d'onduleur (4-3, 4-4) est configurée pour générer la seconde tension alternative haute fréquence.

Fig. 1

Fig. 2

Fig. 3

Fig. 4

EP 4 545 029 B1

Fig. 7

Fig. 8

Fig. 5

Fig. 6

Fig. 9

Fig. 10

Fig. 11

Fig. 12

EP 4 545 029 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3912580 A1

- US 2022370115 A1